# EUROPEAN PATENT SPECIFICATION

(11) **EP 3 088 048 B1**
(45) Date of publication and mention of the grant of the patent: **21.02.2018**
(21) Application number: 14879069.4
(22) Date of filing: 16.01.2014
(51) Int. Cl.: A61N 5/10

(54) **MULTI-LEAF COLLIMATOR AND RADIATION THERAPY DEVICE**
MEHRBLATTKOLLIMATOR UND STRAHLENTHERAPIEVORRICHTUNG
COLLIMATEUR MULTILAMES ET DISPOSITIF DE RADIOTHÉRAPIE

(43) Date of publication of application: 02.11.2016
(73) Proprietor: Hitachi, Ltd., Tokyo 100-8280 (JP)
(72) Inventor: ARAI Satoshi, Tokyo 108-8215 (JP)
(74) Representative: Strehl Schübel-Hopf & Partner
(86) International application number: PCT/JP2014/050702
(87) International publication number: WO 2015/107660

(56) References cited:
- EP-A2- 0 314 231
- JP-A- H01 146 564
- JP-A- H11 216 197
- JP-A- 2006 081 585
- JP-A- 2010 104 452
- JP-B2- 4 436 343
- JP-U- H01 161 012
- US-A1- 2007 176 126
- US-A1- 2012 043 482
- US-A1- 2012 068 087
- US-B1- 7 386 099

## Description

### [Technical Field]

The present invention relates to a multi-leaf collimator which limits a radiation field of radiation radiated to perform radiation therapy and a radiation therapy device using the same.

### [Background Art]

Radiation therapy in which an affected body area is irradiated with radiation is one way of treating tumors. In radiation therapy, the affected body area needs to be efficiently irradiated with radiation while an amount (dose) of radiation with which the patient is irradiated is kept as low as possible.

Consequently, a multi-leaf collimator limit a radiation field, which is an area or a shape in which radiation is irradiated, is being used in a radiation irradiation device.

The multi-leaf collimator includes a plurality of leaves in the shape of thin plates. The leaves are disposed in parallel within a frame at predetermined intervals in the thickness direction of each plate in the multi-leaf collimator. In addition, each of the leaves can be moved by a driving mechanism and is individually advanced and retracted within an irradiation range. When the leaves are disposed in the irradiation range, radiation is shielded by the leaves and the radiation field is limited. That is, a radiation field appropriate for each patient can be formed by limiting the radiation field in this way.

High position precision of the leaves mentioned above is required within the irradiation area. Thus, there are cases in which the driving mechanism detects an actual position of a leaf which has been advanced and retracted to provide the detected result as feedback to a control system of a servomotor.

For example, in Japanese Patent Publication No. 4436343, a constitution in which a protrusion serving as a target is provided on each leaf as a marker is disclosed. In Japanese Patent Publication No. 4436343, a position of the protrusion is identified by capturing the protrusion with a camera and processing the image of the protrusion.

In addition, providing a ruby which emits light when irradiated with light on a leaf as a marker is disclosed in Japanese Patent Publication No. 5143761. In Japanese Patent Publication No. 5143761, a position of a marker is identified by capturing a light-emitting marker with a camera.

US 2007/176126 A1 disclosed a multi-leaf collimator with leaf blocks movable in the direction of the radiation field and having pattern images drawn along the direction of movement on a predetermined surface, and detection part acquiring an image of fixed-point via fixed-point observation in the direction of that predetermined surface and for detecting displacement of the leaf blocks based on the arrangement locations of the pattern images existing in this image of fixed-point.

US 2012/068087 A1 discloses a multi-leaf collimator, the leaves of which are designed to provide for a high step resolution in the projected radiation energy shape.

US 2012/043482 A1 discloses a multi-leaf collimator, the leaves of which are arranged adjacent one another. Leaves of each pair are disposed in an opposed relationship and longitudinally movable relative to each other in a first direction. Leaves of each pair in a further set of leaves are disposed in an opposed relationship and longitudinally movable relative to each other in a second direction generally parallel to the first direction. The first and second set of pairs of leaves are disposed in different planes.

The collimator according to US 7,386,099 B1 comprises a plurality of leaves and guiding members for guiding a movement of the leaves. A pressing unit causing a press contact between the leaves and the guiding members, wherein the pressing unit comprises pressing members which are at least configured to allow for a rolling press contact between the pressing members and the leaves.

EP 0 314 231 A2 discloses a collimating arrangement with a multi-leaf collimator which represents the closest prior art.

### [Summary of Invention]

### [Technical Problem]

However, a resolution of the shape of an irradiation field depends on a thickness of each of the leaves in the multi-leaf collimator described above. To improve the resolution of the irradiation field, each of the leaves should be thinned and the number of leaves should be increased.

However, when the leaves are thinned, markers of the plurality of leaves aligned in a thickness direction are closely and densely disposed. Thus, each of the markers may not be independently identified when the markers are captured with a camera and images of the markers are processed, and positions of the leaves may not be accurately detected.

With respect to this, improving a resolution of the camera to allow the individual markers to be more accurately identified may be considered. However, since data volumes of the captured images increase, image processing may take a long time and it may be difficult to detect the positions of the leaves in real time.

The present invention has been devised in consideration of the circumstances above and is directed to providing a multi-leaf collimator in which a marker provided on a leaf can be more accurately identified and a position of the leaf can be promptly detected with a high precision and radiation therapy device using the same.

### [Solution to Problem]

This object is solved by a multi-leaf collimator with the features of claim 1. Preferred embodiments of the multi-leaf collimator and a radiation therapy device with the multi-leaf collimator are set out in the other claims.

The present invention employs the following means to solve the problem above.

According to a first aspect of the present invention, a multi-leaf collimator is a multi-leaf collimator which limits a radiation field, and includes a plurality of leaves aligned in a thickness direction of the leaf, a driving mechanism which advances and retracts each of the plurality of leaves with respect to the radiation, and an identification mark provided on each of the plurality of leaves and identifiable at least from a direction perpendicular to both an advancing and retracting direction of the leaves and the thickness direction of the leaf, wherein, when the plurality of leaves are disposed in a preset reference position, gaps between adjacent identification marks in the thickness direction are formed to be larger than gaps between adjacent leaves in the thickness direction.

According to the present invention, in the multi-leaf collimator according to the first aspect, a width of the identification mark in the thickness direction may be formed to be smaller than a thickness of the leaf including the identification mark.

According to the present invention, the plurality of leaves is constituted of various types of leaves having different thicknesses, and the number of types of widths of the identification mark is the same as or less than the number of types of thicknesses of the leaves.

According to the present invention, in the multi-leaf collimator according to any one of the first to third aspects, the identification marks of the adjacent leaves in the thickness direction may be disposed at different positions in the advancing and retracting direction.

According to a further aspect of the present invention, in the multi-leaf collimator according to any one of the first to fourth aspects, the identification mark may be formed at an end surface in a direction perpendicular to both the advancing and retracting direction and the thickness direction of the leaf.

According to a further aspect of the present invention, in the multi-leaf collimator according to the fifth aspect, the end surface may include at least one of a convex portion and a groove portion, and the identification mark may be formed on at least one of the convex portion and the groove portion.

According to a further aspect of the present invention, a radiation therapy device includes a multi-leaf collimator according to any one of the first to sixth aspects, a mark position detection unit which detects a position of the identification mark, a control unit which controls the driving mechanism based on the position of the identification mark detected by the mark position detection unit, and a radiation irradiation device which radiates radiation.

### [Advantageous Effects of Invention]

According to a multi-leaf collimator and a radiation therapy device of the present invention, identification of an identification mark provided on a leaf can be more ensured and a position of the leaf can be promptly detected with high precision.

### [Brief Description of Drawings]

Fig. 1 is a view illustrating a functional constitution of a radiation therapy system according to a first embodiment of the present invention.
Fig. 2 is a perspective view illustrating a schematic constitution of a radiation therapy device which constitutes the radiation therapy system.
Fig. 3 is a cross-sectional view illustrating a radiation irradiation device which constitutes the radiation therapy device.
Fig. 4 is a perspective view illustrating an exterior of a multi-leaf collimator which constitutes part of the radiation irradiation device.
Fig. 5 is a cross-sectional view of the multi-leaf collimator in a width direction.
Fig. 6 is a cross-sectional view of the multi-leaf collimator in a direction perpendicular to a plate thickness direction of a leaf.
Fig. 7 is a perspective view illustrating a leaf and a driving device which drives the leaf.
Fig. 8 is a perspective view illustrating a constitution of the driving device.
Fig. 9 is a perspective view illustrating a constitution of an identification mark provided on each of the plurality of leaves.
Fig. 10 is a plan view illustrating a relation between positions of identification marks on the plurality of leaves.
Fig. 11 is a view illustrating a schematic constitution of a mark position detection unit for detecting a position of an identification mark formed on the leaf.
Fig. 12 is a plan view illustrating identification marks provided on a plurality of leaves according to a first modified example of the first embodiment.
Fig. 13 is a plan view illustrating identification marks provided on a plurality of leaves according to a second modified example of the first embodiment.
Fig. 14 is a perspective view illustrating a constitution of an identification mark provided on each leaf according to a second embodiment of the present invention.
Fig. 15 is a plan view illustrating a constitution of an identification mark provided on each leaf according to a third embodiment of the present invention.
Fig. 16 is a plan view illustrating identification marks provided on a plurality of leaves according to a first modified example of the third embodiment.
Fig. 17 is a perspective view illustrating a constitution of a leaf including an identification mark according to a first modified example of the second embodiment.
Fig. 18 is a perspective view illustrating a constitution of a leaf including an identification mark according to a second modified example of the second embodiment.
Fig. 19 is a perspective view illustrating a constitution of a leaf including an identification mark according to a third modified example of the second embodiment.
Fig. 20 is a perspective view illustrating a constitution of a leaf including an identification mark according to a fourth modified example of the second embodiment.
Fig. 21 is a perspective view illustrating a constitution of a leaf including an identification mark according to a fifth modified example of the second embodiment.
Fig. 22 is a perspective view illustrating a constitution of a leaf including an identification mark according to a sixth modified example of the second embodiment.

### [Description of Embodiments]

Hereinafter, a multi-leaf collimator and a radiation therapy device according to an embodiment of the present invention will be described with reference to the drawings.

### (First embodiment)

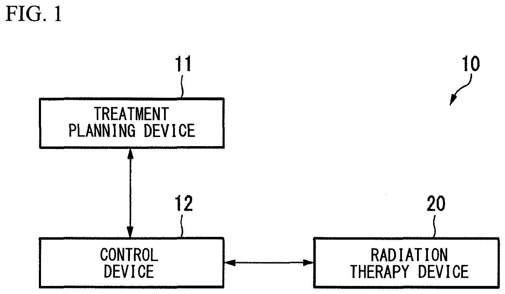

Fig. 1 is a view illustrating a functional constitution of a radiation therapy system 10 according to a first embodiment of the present invention.

As illustrated in Fig. 1, the radiation therapy system 10 includes a treatment planning device 11, a control device (control unit) 12, and a radiation therapy device 20.

The treatment planning device 11 is a device to which properties of radiation with which a patient will be irradiated (intensity, time, angle, position, irradiation area, etc. of radiation with which the patient will be irradiated) are preset according to details of radiation therapy carried out on the patient are input from the outside. The treatment planning device 11 outputs values of various types of parameters for controlling in order to radiate radiation corresponding to the properties of radiation input to the control device 12.

The control device 12 controls an operation of the radiation therapy device 20 based on the values of the various types of parameters generated by the treatment planning device 11. The control device 12 is a computer device such as a personal computer which executes processing on the basis of a predetermined program. The control device 12 is connected to the radiation therapy device 20 via a wireless or wired communication line so as to be capable of two-way transmission of information.

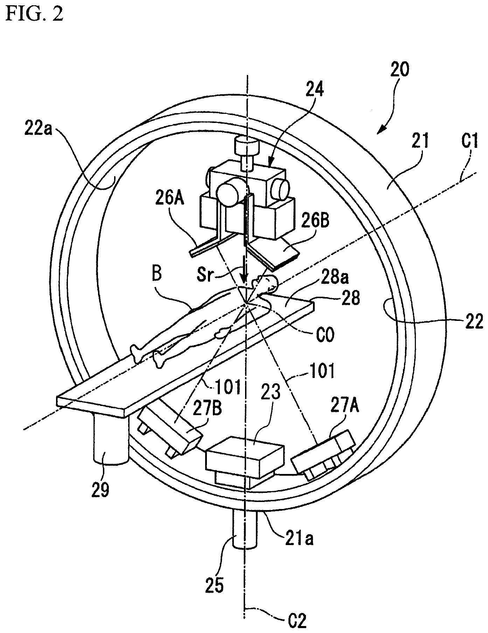

Fig. 2 is a perspective view illustrating a schematic constitution of the radiation therapy device 20 which constitutes the radiation therapy system 10.

As illustrated in Fig. 2, the radiation therapy device 20 includes a ring frame 21, a travelling gantry 22, and a radiation irradiation device 24.

The ring frame 21 is formed in a cylindrical shape having a circular cross-section. The ring frame 21 is disposed so that a central axis C1 substantially directs a horizontal direction. The ring frame 21 includes a rotation shaft 25 which extends downward integrally formed with an outer circumferential surface of a lower end portion 21a thereof. The rotation shaft 25 is supported by a base (not illustrated) in a state in which it is capable of rotating about a central axis C2 thereof. The rotation shaft 25 is driven to rotate by a rotation driving mechanism (not illustrated). That is, the ring frame 21 rotates about a perpendicular axis by the rotation shaft 25 rotated by the rotation driving mechanism.

The travelling gantry 22 is formed in a cylindrical shape having a circular cross-section. The travelling gantry 22 is disposed at an inner circumferential side of the ring frame 21. The travelling gantry 22 is supported by the ring frame 21 and is rotatable along an inner circumferential surface of the ring frame 21. In other words, the ring-shaped travelling gantry 22 is capable of rotating about the central axis C1 which extends in the horizontal direction. The travelling gantry 22 is rotated in the circumferential direction by a gantry driving mechanism (not illustrated).

The radiation irradiation device 24 is controlled by the control device 12 (refer to Fig. 1) and radiates therapeutic radiation Sr. The radiation irradiation device 24 is supported by an inner circumferential surface 22a of the travelling gantry 22. The therapeutic radiation Sr radiated from the radiation irradiation device 24 is adjusted to pass through an isocenter C0 which is an intersection point between the central axis C2 of the rotational movement of the ring frame 21 and the central axis C1 of the rotational movement of the travelling gantry 22.

In this manner, by the radiation irradiation device 24 supported by the travelling gantry 22, the therapeutic radiation Sr is radiated to always pass through the isocenter C0 regardless of the rotational movement of the ring frame 21 about the central axis C2 and the rotational movement of the travelling gantry 22 about the central axis C1.

The radiation therapy device 20 further includes a sensor array 23. The sensor array 23 receives the therapeutic radiation Sr that has been radiated by the radiation irradiation device 24 and transmitted through a subject near the isocenter C0 and generates a transmission image of the subject. A flat panel detector (FPD), an X-ray image intensifier (II), etc. may be used as the sensor array 23.

In addition, the radiation therapy device 20 includes diagnostic X-ray sources 26A and 26B and sensor arrays 27A and 27B.

The diagnostic X-ray sources 26A and 26B are disposed at an inner circumferential side of the travelling gantry 22. The diagnostic X-ray sources 26A and 26B are disposed at both sides of the center of the radiation therapy device 20 (in other words, the central axis C2 of the rotational movement of the ring frame 21), along to the circumferential direction of the ring frame 21 interposed therebetween. The diagnostic X-ray sources 26A and 26B are controlled by the control device 12 and radiate diagnostic X-rays 101 toward the isocenter C0. The diagnostic X-rays 101 are a conical beam diffused in a conical shape from one point of the diagnostic X-ray sources 26A and 26B.

The sensor arrays 27A and 27B are supported by the inner circumferential surface 22a of the travelling gantry 22. The sensor arrays 27A and 27B are disposed to face the diagnostic X-ray sources 26A and 26B with the isocenter C0 interposed therebetween. The sensor arrays 27A and 27B receive the diagnostic X-rays 101radiated from the diagnostic X-ray sources 26A and 26B and transmitted through a subject near the isocenter C0 and generate a transmission image of the subject. A flat panel detector (FPD), an X-ray image intensifier (II), etc. may be, for example, used as the sensor arrays 27A and 27B.

The radiation therapy device 20 further includes a couch 28 and a couch driving device 29. The couch 28 includes an upper surface 28a on which a patient B being treated by the radiation therapy system 10 lies down.

The couch driving device 29 is controlled by the control device 12 and moves the couch 28. The couch driving device 29 is supported by a base (not illustrated).

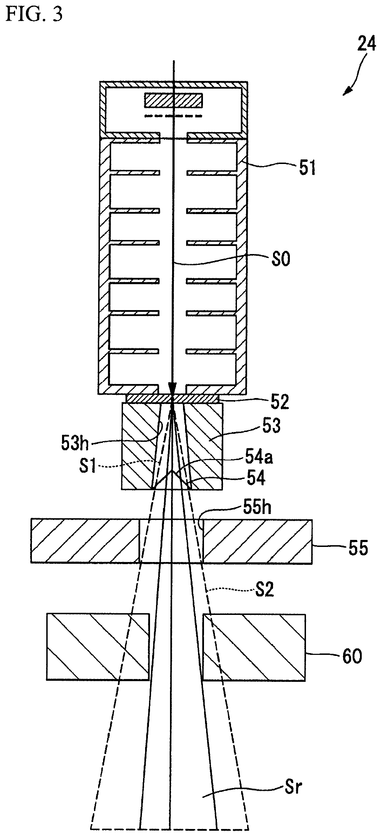

Fig. 3 is a cross-sectional view illustrating the radiation irradiation device 24 which constitutes the radiation therapy device 20.

As illustrated in Fig. 3, the radiation irradiation device 24 includes an electron beam acceleration device 51, an X-ray target 52, a first collimator 53, a flattening filter 54, a second collimator 55, and a multi-leaf collimator 60.

The electron beam acceleration device 51 irradiates the X-ray target 52 with an electron beam S0 generated by accelerating electrons.

The X-ray target 52 is formed of tungsten, a tungsten alloy, etc. The X-ray target 52 emits radiation S1 when irradiated with the electron beam S0.

The first collimator 53 shields part of the radiation S1 so that portions other than desired portions are not irradiated with the radiation S1. The first collimator 53 includes a through-hole 53h though which the radiation S1 radiated from the X-ray target 52 passes. The first collimator 53 is formed of lead, tungsten, etc.

The flattening filter 54 is a filter which substantially evenly distributes dose of the radiation S1 on a plane which is perpendicular to a radiation direction of the radiation S1. The flattening filter 54 is formed of aluminum and the like. The flattening filter 54 is disposed at an outlet side of the through-hole 53h of the first collimator 53. The flattening filter 54 has a protrusion 54a in a substantially conical shape disposed at a side facing the X-ray target 52. The shape of the protrusion 54a is designed so that the dose of the radiation S1 is substantially evenly distributed on the plane which is perpendicular to the radiation direction of the radiation S1.

The second collimator 55 shields some of the radiation S1. The second collimator 55 has a through-hole 55h at a central portion thereof. The second collimator 55 allows radiation S2 to pass therethrough only at the through-hole 55h. The second collimator 55 is formed of lead, tungsten, etc.

By passing through the first collimator 53, the flattening filter 54, and the second collimator 55 described above, some of the radiation S2 having an evenly distributed intensity is further shielded by the multi-leaf collimator 60. The multi-leaf collimator 60 is controlled by the control device 12 and limits a radiation field of the radiation S2. The multi-leaf collimator 60 generates the therapeutic radiation Sr according to properties of radiation which should be radiated to a patient.

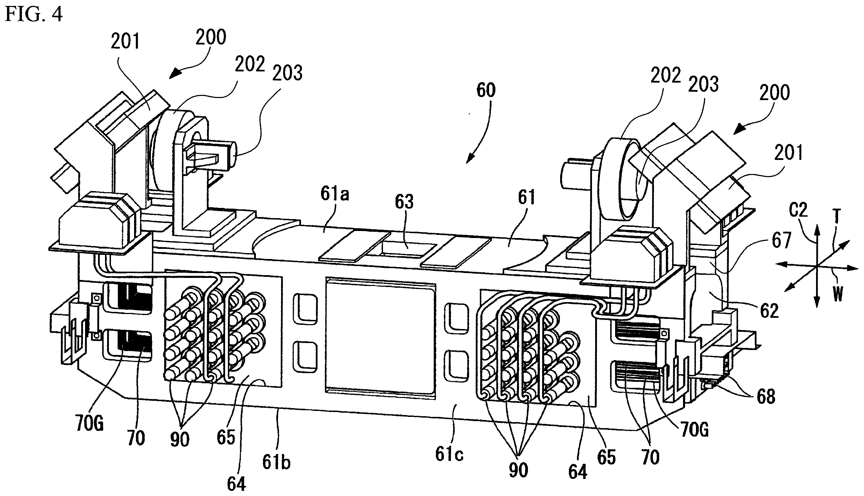

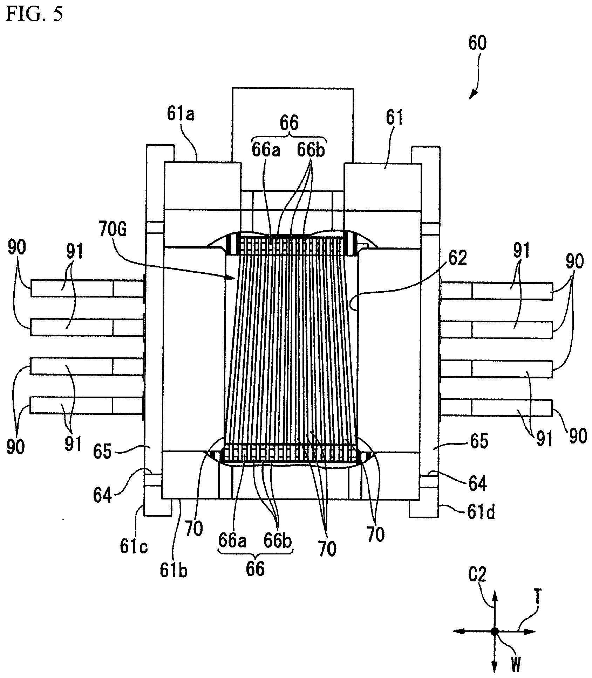

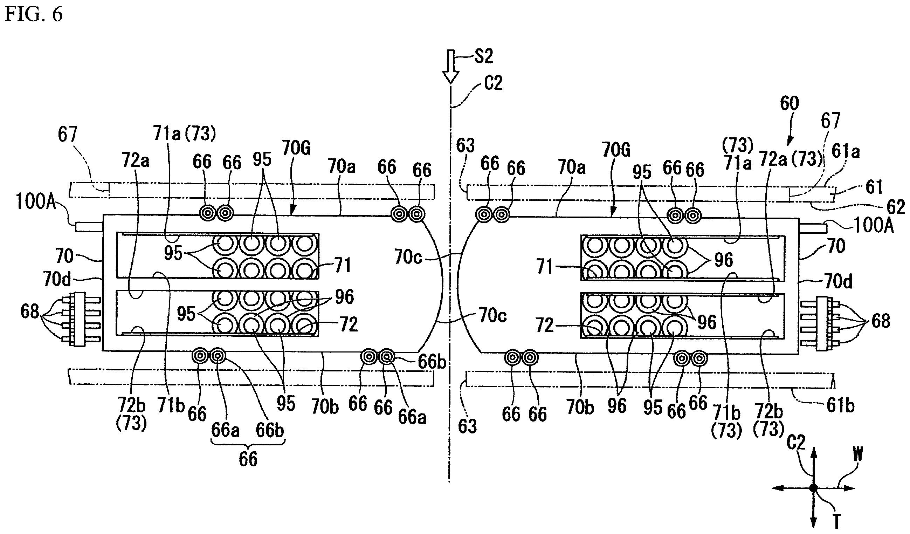

Fig. 4 is a perspective view illustrating an exterior of the multi-leaf collimator 60 which constitutes part of the radiation irradiation device 24. Fig. 5 is a cross-sectional view of the multi-leaf collimator 60 in a width direction. Fig. 6 is a cross-sectional view of the multi-leaf collimator 60 in its direction perpendicular to a second direction which is a thickness direction of a leaf 70 (hereinafter, the second direction will be referred to as plate thickness direction T).

As illustrated in Figs. 4 to 6, the multi-leaf collimator 60 includes a frame 61, a plurality of leaves 70, and a driving device (driving mechanism) 90.

The frame 61 is formed substantially in a rectangular parallelepiped shape which is long in one direction. The frame 61 is disposed such that a first direction which is a longitudinal direction thereof (hereinafter, the first direction will be referred to as a width direction W) is perpendicular to a radiation beam axis of the radiation irradiation device 24. A hollow leaf accommodation unit 62 is continuously formed in the width direction W in the frame 61.

In the frame 61, openings 63 which penetrate an outer circumferential side of the frame 61 and the leaf accommodation unit 62 are formed at an upper surface portion 61a at a side facing the radiation irradiation device 24 and a lower surface portion 61b which is at the opposite side (only the opening 63 at the upper surface portion 61a is illustrated in Fig. 4). The openings 63 are formed at central portions of the upper surface portion 61a and the lower surface portion 61b in the width direction W.

As illustrated in Figs. 4 and 5, in the frame 61, rectangular openings 64 and 64 are respectively formed at both side surface portions 61c and 61d which are perpendicular to the upper surface portion 61a and the lower surface portion 61b. The opening 64 of the side surface portion 61c and the opening 64 of the side surface portion 61d are formed to be symmetrical with respect to a virtual plane disposed at the center between the side surface portion 61c and the side surface portion 61d. Rectangular base plates 65 are mounted on the openings 64.

The leaf 70 is formed in the shape of a substantially rectangular plate. The leaf 70 is formed of, for example, tungsten, a tungsten alloy, etc. through which the radiation S2 is not transmitted.

As illustrated in Fig. 5, a plurality of leaves 70 are arranged at predetermined intervals in the plate thickness direction T. A leaf group 70G is constituted by the plurality of leaves 70. In the embodiment, the leaf group 70G is constituted, for example, by thirty leaves 70. As illustrated in Figs. 4 and 6, a pair of leaf groups 70G are disposed in the leaf accommodation unit 62 in the frame 61 to face each other with a central portion in the width direction W of the frame 61 interposed therebetween.

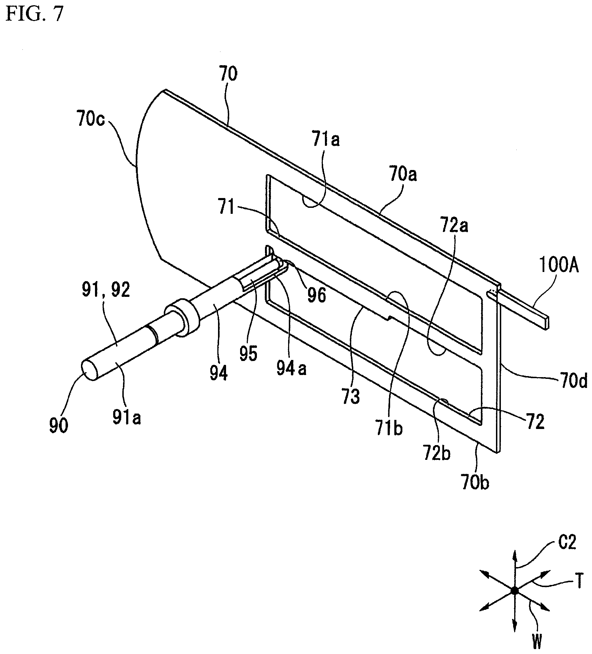

Fig. 7 is a perspective view illustrating the leaf 70 and the driving device 90 which drives the leaf 70.

As illustrated in Figs. 6 and 7, the leaf 70 includes a straight upper edge portion (end surface) 70a and a straight lower edge portion 70b formed parallel to each other. As illustrated in Fig. 6, the upper edge portion 70a is disposed to face the upper surface portion 61a while having a gap therebetween in the leaf accommodation unit 62. Likewise, the lower edge portion 70b is disposed to face the lower surface portion 61b with a gap therebetween in the leaf accommodation part 62.

The leaf 70 includes a front edge portion 70c at a side facing the central portion in the width direction W of the frame 61 and formed in the shape of an arc in the leaf accommodation unit 62. In addition, the leaf 70 includes a rear edge portion 70d facing the outside in the width direction W of the frame 61 formed in a straight shape perpendicular to the upper edge portion 70a and a lower edge portion 70b in the leaf accommodation unit 62.

The pair of leaf groups 70G, 70G disposed to face each other with the central portion in the width direction W interposed therebetween in the leaf accommodation unit 62 are disposed so that the front edge portion 70c of each of the leaves faces an area between the opening 63 of the upper surface portion 61a and the opening 63 of the lower surface portion 61b of the frame 61.

Each of the leaves 70 includes slits 71 and 72 that penetrate in the plate thickness direction T. The slits 71 and 72 are continuously formed in a direction in which the front edge portion 70c and the rear edge portion 70d of each of the leaves 70 are connected to each other, i.e., the width direction W. The slits 71 and 72 are arranged at a predetermined interval in a direction in which the upper edge portion 70a and the lower edge portion 70b of the leaf 70 are connected to each other. The slits 71 and 72 are formed closer to the rear edge portion 70d than to the front edge portion 70c to prevent the radiation S2 incident from the opening 63 of the upper surface portion 61a of the frame 61 to an inside of the leaf accommodation unit 62 of the frame 61 from being irradiated thereto.

Each of the leaves 70 includes a rack gear 73 continuously formed in the width direction W on at least one of upper side portions 71a and 72a and lower side portions 71b and 72b of the slits 71 and 72. Here, in the leaf groups 70G of the embodiment, the rack gears 73 of the leaves 70 and 70 adjacent in a direction in which the plurality of leaves 70 are arranged are formed at different sides among the upper side portions 71a and 72a and the lower side portions 71b and 72b of the slits 71 and 72. In this way, pinion gears 96 which interlock with the rack gears 73 can be prevented from interfering with each other between the leaves 70 and 70 adjacent in the plate thickness direction T.

The plurality of leaves 70 which constitute each of the leaf groups 70G are supported by the frame 61. The frame 61 supports the leaf 70 so that the leaf 70 is capable of advancing and retracting in the width direction W which is perpendicular to the plate thickness direction T. The frame 61 includes a plurality of slide support members 66. The plurality of slide support members 66 are disposed at predetermined intervals in the width direction W at an upper portion and a lower portion of each of the leaf groups 70G. In the embodiment, two slide support members 66 are disposed at each of the central portion side and the outer circumferential side of the frame 61 in the width direction W, i.e., a total of four slide support members 66 which guide movement of the leaf 70 are provided at the upper portion and the lower portion of each of the leaf groups 70G.

As illustrated in Figs. 5 and 6, each of the slide support members 66 includes a shaft 66a fixed to the frame 61 and a plurality of support rollers 66b rotatably mounted on the shaft 66a. The plurality of support rollers 66b are disposed at positions corresponding to the plurality of leaves 70 which constitute the leaf group 70G. The support rollers 66b are constituted to perform a rotation in a direction in which the upper edge portion 70a and the lower edge portion 70b of the leaf 70 extend.

As illustrated in Fig. 6, the support rollers 66b of the slide support members 66 come in contact with the upper edge portion 70a and the lower edge portion 70b of each of the leaves 70. At least two support rollers 66b coming in contact with the upper edge portion 70a and at least two support rollers 66b coming in contact with the lower edge portion 70b are provided.

That is, each of the leaves 70 is supported by the slide support members 66 in the frame 61 in a state in which it can individually advance and retract in the width direction W.

Here, in the leaf group 70G described above, the support rollers 66b of different slide support members 66 among the plurality of slide support members 66 described above may come in contact with the leaves 70 and 70 adjacent in the plate thickness direction T. In this way, the support rollers 66b can be prevented from interfering with each other between the adjacent leaves 70 and 70.

The frame 61 includes a stopper 68 which regulates a movement amount of each of the leaves 70 toward the rear edge portion 70d in the width direction W.

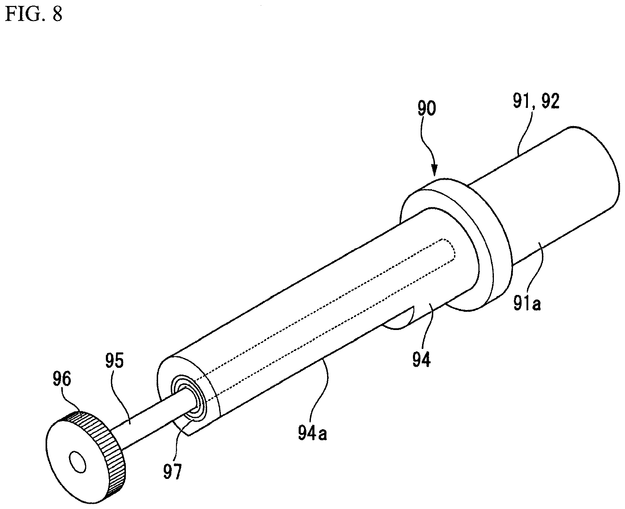

Fig. 8 is a perspective view illustrating a constitution of the driving device 90.

As illustrated in Figs. 7 and 8, the driving device 90 is provided to correspond to each of the plurality of leaves 70. The driving device 90 includes a motor 91, a shaft 95, and the pinion gear 96.

The motor 91 is connected to a proximal end portion of the shaft 95. The motor 91 drives the shaft 95 to rotate about the axis thereof.

Here, as illustrated in Fig. 5, the motor 91 is supported by the base plates 65 provided along the side surface portions 61c and 61d of the frame 61.

The motor 91 of the driving device 90, which drives one half of the leaves 70 at a side close to the side surface portion 61c among the plurality of leaves 70 which constitute the leaf group 70G, is supported by the base plate 65 provided at the side surface portion 61c at one side of the frame 61. The motor 91 of the driving device 90, which drives the other half of the leaves 70 at a side close to the side surface portion 61d among the plurality of leaves 70 which constitute the leaf group 70G, is supported by the base plate 65 provided at the side surface portion 61d at the other side of the frame 61.

As illustrated in Fig. 7, the shaft 95 extends in the plate thickness direction T of the leaf 70. In addition, as illustrated in Figs. 6 and 7, the shaft 95 is inserted into the slit 71 or 72 of the plurality of leaves 70 of the leaf group 70G.

In addition, as illustrated in Figs. 7 and 8, the pinion gear 96 is mounted on a distal end portion of the shaft 95. The pinion gear 96 is interlocked with the rack gear 73 formed on any one of the upper side portions 71a and 72a and the lower side portions 71b and 72b of the slits 71 and 72 which are part of the leaf 70.

The driving device 90 further includes a rotary encoder 92 and a cover 94.

The rotary encoder 92 measures a rotational amount of the shaft 95 and outputs the measurement result to the control device 12.

The cover 94 is formed in the shape of a hollow tube. The cover 94 is integrally provided with a housing 91a of the motor 91. A bearing 97 is provided at an end portion of the cover 94 which is the opposite side from the motor 91. In addition, the shaft 95 is inserted into the cover 94. The shaft 95 is rotatably supported by the bearing 97. That is, the cover 94 supports the shaft 95 by the bearing 97 at a position spaced apart in a direction in which the shaft 95 extends from the motor 91. In this way, the shaft 95 is prevented from being deformed due to a self-weight and the like, and the pinion gear 96 is ensured to be interlocked with teeth of the rack gear 73 even when the motor 91 and the leaf 70 are spaced apart from each other.

A notch 94a is formed at the cover 94 partly in the circumferential direction.

The notch 94a prevents the leaf 70, having the rack gear 73 with which the pinion gear 96 of the shaft 95 inserted into the cover 94 is interlocked, from interfering with another leaf 70 disposed closer to the motor 91.

In the driving device 90, the motor 91 is driven by the control of the control device 12 and rotates the shaft 95. When the shaft 95 rotates, the pinion gear 96 rotates together with the shaft 95, and the rotary force is transmitted to the rack gear 73. Then, the leaf 70 having the rack gear 73 is displaced in the advancing and retracting direction which is the width direction W.

In this way, with respect to each of the pair of leaf groups 70G, when each of the leaves 70 which constitute the leaf groups 70G is advanced and retracted in the width direction W, some of the radiation S2 incident from the opening 63 of the upper surface portion 61a of the frame 61 is shielded by the leaf 70 of the leaf groups 70G at both sides. That is, the therapeutic radiation Sr limited to a predetermined shape of a radiation field is generated by the multi-leaf collimator 60.

Treatment is performed as follows in the radiation therapy system 10 described above.

First, a user fixes a patient B to the couch 28 of the radiation therapy device 20 so that the patient B is in a posture indicated in a treatment plan input to the treatment planning device 11.

The control device 12 operates the rotation driving mechanism (not illustrated) and a gantry driving device (not illustrated). Specifically, the control device 12 rotates the ring frame 21 and the travelling gantry 22 about the central axes C1 and C2 and moves the radiation irradiation device 24 so that an affected body area of the patient B is irradiated with the therapeutic radiation Sr at an irradiation angle indicated in the treatment plan. In addition, the control device 12 advances and retracts each of the leaves 70 by the driving device 90 so that the limited shape of the irradiation field of the therapeutic radiation Sr is changed to a shape indicated in the treatment plan input to the treatment planning device 11 in the multi-leaf collimator 60.

Then, the control device 12 uses the radiation irradiation device 24 to irradiate the affected body area of the patient B with the therapeutic radiation Sr of a dosage indicated in the treatment plan input to the treatment planning device 11.

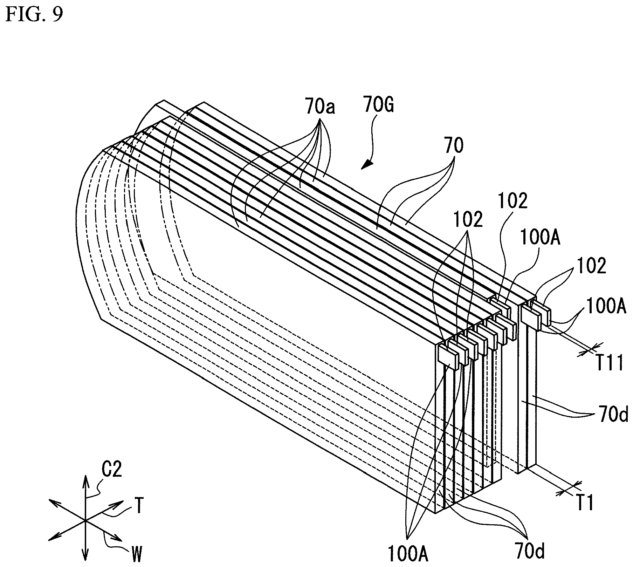

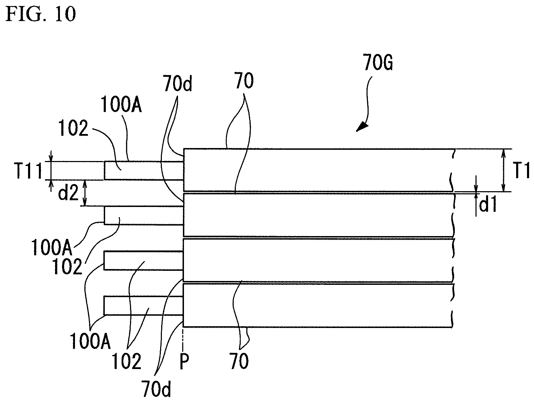

Fig. 9 is a perspective view illustrating a constitution of an identification mark 102 provided on each of the plurality of leaves 70. Fig. 10 is a plan view illustrating a relation between positions of identification marks 102 on the plurality of leaves 70.

As illustrated in Figs. 6, 7, and 9, a marker unit 100A is provided at each of the leaves 70 constituting each of the leaf groups 70G of the multi-leaf collimator 60. The marker units 100A are provided for monitoring positions of the leaves 70 advanced and retracted by the driving device 90.

As illustrated in Fig. 9, the marker unit 100A is formed to protrude from the rear edge portion 70d of the leaf 70 to an outside in the width direction W in which the upper edge portion 70a extends. A plate thickness T11 of the marker unit 100A is formed to be smaller than a plate thickness T1 of the leaf 70.

In the embodiment, the marker unit 100A extends from a central portion in the plate thickness direction T of the leaf 70 to the outside in the width direction W.

The identification mark 102 is formed on the whole upper surface of the marker unit 100A by coating, and the like so that the marker unit 100A is identifiable at least from an upper portion perpendicular to both the advancing and retracting direction of the leaf 70 and the plate thickness direction T of the leaf 70. Here, in the embodiment, the identification mark 102 is formed by, for example, coating the whole upper surface of the marker unit 100A.

The marker unit 100A is exposed upward from an opening 67 (refer to Figs. 4 and 6) formed above the leaf accommodation unit 62 at an upper surface of the frame 61 described above.

As illustrated in Fig. 10, when the plurality of leaves 70 each having the marker unit 100A are disposed in a predetermined reference position, gaps d2 between adjacent identification marks 102 on the marker units 100A of leaves 70 in the plate thickness direction T are made to be larger than gaps d1 between adjacent leaves 70 in the plate thickness direction T. Here, the reference position is, for example, a retracting end position P at which the rear edge portion 70d of each leaf 70 comes in contact with the stopper 68 (refer to Figs. 4 and 6).

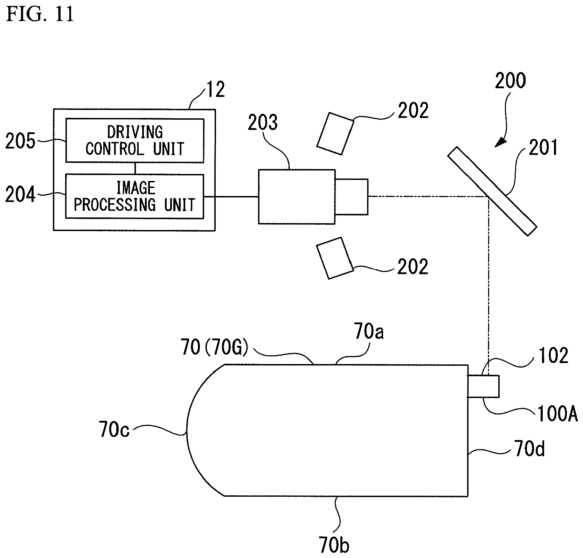

Fig. 11 is a view illustrating a schematic constitution of a mark position detection unit 200 for detecting a position of the identification mark 102 on the marker unit 100A formed on the leaf 70.

As illustrated in Figs. 4 and 11, the radiation therapy device 20 includes the mark position detection unit 200. The mark position detection unit 200 detects a position of the identification mark 102 on the marker unit 100A formed on the leaf 70.

The mark position detection unit 200 includes a mirror 201, illumination 202, a camera 203, and an image processing unit 204. The mirror 201, the illumination 202, and the camera 203 are provided on the frame 61. The image processing unit 204 is provided as one function of the control device 12 by cooperation between the computer device and a computer program which constitute the control device 12.

The mirror 201 is disposed above the rear edge portion 70d of the plurality of leaves 70 which constitute the leaf group 70G.

The illumination 202 emits illumination light to the rear edge portion 70d of the plurality of leaves 70 through the mirror 201.

The camera 203 captures the rear edge portion 70d of the plurality of leaves 70 through the mirror 201. The camera 203 outputs captured image data to the image processing unit 204.

The image processing unit 204 executes predetermined image processing of the image data output from the camera 203. The image processing unit 204 identifies the identification mark 102 in the image and specifies the position thereof. Here, master data such as a shape, a size, etc. of the identification mark 102 is preregistered in the image processing unit 204 to identify the identification mark 102.

In addition, an image processing method of the image processing unit 204 is not limited at all, and a known image processing method such as a pattern matching method may be used.

The image processing unit 204 performs identification and position specification with respect to identification marks 102 of all of the leaves 70 included in the image by the image processing. In this way, a position of each of the leaves 70 in the advancing and retracting direction is detected. The image processing unit 204 transmits the detected result to a driving control unit 205.

The driving control unit 205 is provided in the control device 12. The driving control unit 205 executes feedback control of the driving device 90 based on the position of each of the leaves 70 and compensates a control value of the motor 91 as needed.

Consequently, according to the multi-leaf collimator 60 and the radiation therapy device 20 of the first embodiment described above, because the plate thickness T11 of the marker unit 100A is formed to be smaller than the plate thickness T1 of the leaf 70, the gaps d2 between adjacent identification marks 102 on the marker units 100A of the leaves 70 may be formed to be larger than the gaps d1 between adjacent leaves 70 in the plate thickness direction T. In this way, when the identification marks 102 formed on the marker units 100A are captured and images thereof are processed, identification of the identification marks 102 provided on the leaves 70 can be ensured and positions of the leaves 70 can be detected with high precision since the identification marks 102 are spaced apart from each other. In addition, the need for improving image resolution of the camera 203 for performing the detection with high precision is low. Thus, a delay of a processing speed is suppressed and prompt processing can be performed.

### (First modified example of the first embodiment)

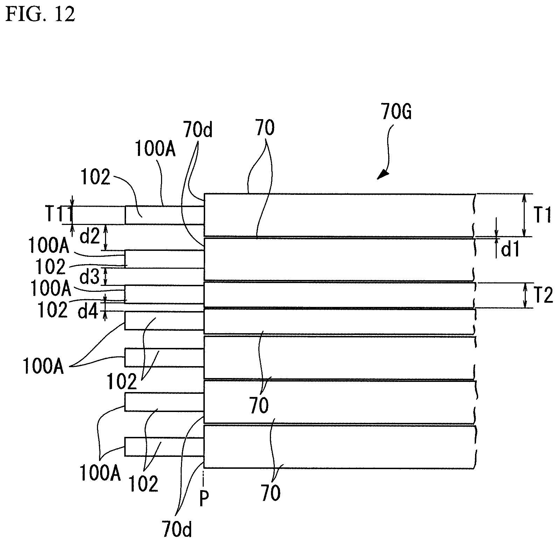

Fig. 12 is a plan view illustrating the marker units 100A provided on the plurality of leaves 70 according to a first modified example of the first embodiment.

A case in which the plate thicknesses of the leaves 70 which constitute the leaf group 70G have only one type has been described as an example in the first embodiment described above. However, the plate thicknesses of the leaves 70 which constitute the leaf group 70G are not limited to the one type. As illustrated in Fig. 12, two or more types of the plate thicknesses may be mixed. In the example of Fig. 12, the leaf group 70G is constituted of two types of the leaves 70 having plate thicknesses T1 and T2, respectively. In more detail, the plate thickness T2 of the leaves 70 disposed at a central side in the plate thickness direction T is formed to be thinner than the plate thickness T1 of the leaves 70 disposed at an outside in the plate thickness direction T in the leaf group 70G.

On the other hand, as in the first embodiment, the plurality of leaves 70 which constitute the leaf group 70G are provided on the marker units 100A at the rear edge portions 70d. The plate thickness T11 of the marker unit 100A in the plate thickness direction T is formed to be smaller than the plate thicknesses T1 and T2 of the leaves 70. The identification mark 102 is formed on an upper surface of each of the marker units 100A.

In addition, when the plurality of leaves 70 which constitute the leaf group 70G are disposed at the retracting end position P which is a predetermined reference position, gaps d2, d3, and d4 between the adjacent identification marks 102 in the plate thickness direction T are formed to be larger than the gaps d1 between adjacent leaves 70 in the plate thickness direction T.

Consequently, according to the first modified example, as in the first embodiment, identification of the identification marks 102 can be ensured and positions of the leaves 70 can be detected with high precision.

### (Second modified example of the first embodiment)

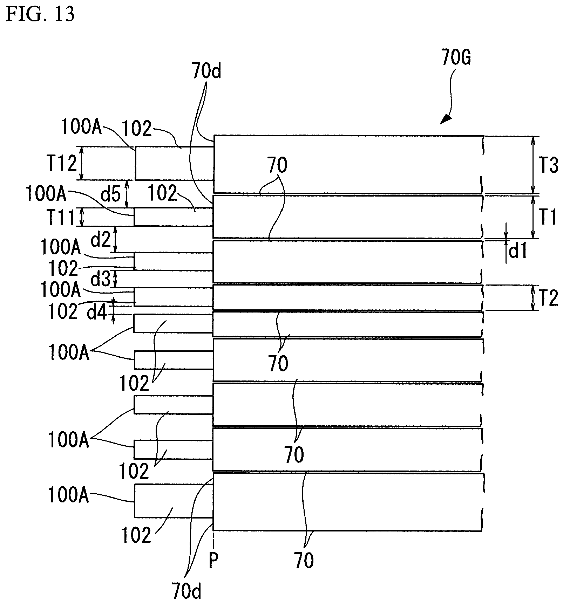

Fig. 13 is a plan view illustrating identification marks 102 provided on the plurality of leaves 70 according to a second modified example of the first embodiment.

In the second modified example illustrated in Fig. 13, the plate thicknesses of the leaves 70 which constitute the leaf group 70G have three types, T1, T2, and T3. The leaves 70 include the marker units 100A having two types of plate thicknesses T11 and T12, and the identification marks 102 are provided on the marker units 100A.

In addition, when the plurality of leaves 70 which constitute the leaf group 70G are disposed at the retracting end position P which is a predetermined reference position, the gaps d2, d3, d4, and d5 between the adjacent identification marks 102 in the plate thickness direction T are formed to be larger than the gaps d1 between adjacent leaves 70 in the plate thickness direction T.

Consequently, according to the second modified example, as in the first embodiment, identification of the identification marks 102 can be ensured and positions of the leaves 70 can be detected with high precision.

In addition, as in the first modified example and the second modified example described above, when the plurality of leaves 70 are constituted of the at least two types of leaves having plate thicknesses T1 and T2 or T1, T2, and T3, respectively, types of the identification marks 102 prestored in the image processing unit 204 for identifying the marker units 100A can be reduced since the marker units 100A formed on the plurality of leaves 70 have fewer number of types of the plate thicknesses T11 and T12 than the number of types of thicknesses of the leaves 70 (equal to or less than the number of types of the plate thicknesses of the leaves 70).

### (Second embodiment)

Next, a multi-leaf collimator and a radiation therapy device according to a second embodiment of the present invention will be described. Since only a constitution of a marker unit 100B is different in the second embodiment to be described below compared to the first embodiment, similar reference signs will be given to parts that are the same as in the first embodiment and overlapping descriptions thereof will be omitted.

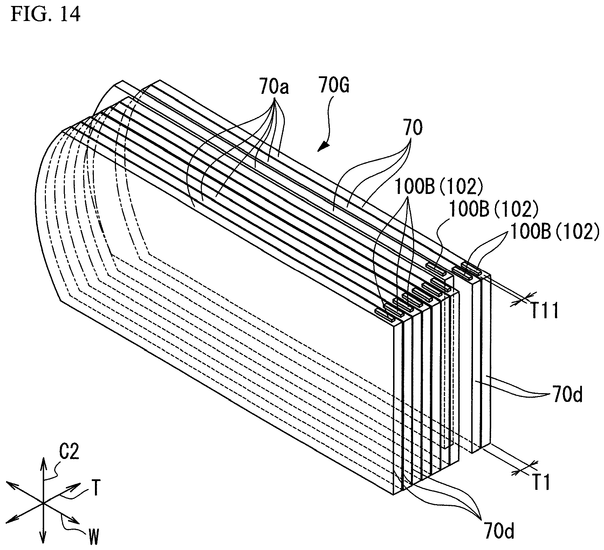

Fig. 14 is a perspective view illustrating a constitution of an identification mark provided on each leaf according to a second embodiment of the present invention.

As illustrated in Fig. 14, the marker unit 100B of the embodiment is integrally formed on each of the upper edge portions 70a of each of the plurality of leaves 70 which constitute the leaf group 70G.

The marker unit 100B is formed to protrude upward from the upper edge portion 70a of the leaf 70. The marker unit 100B is formed in a rectangular parallelepiped shape having a longitudinal direction in the advancing and retracting direction of the leaf 70. The marker unit 100B is formed such that the plate thickness T11 thereof in the plate thickness direction is formed to be smaller than the plate thickness T1 of the leaf 70. In addition, the marker unit 100B is disposed at a central portion of the leaf 70 in the plate thickness direction T.

The identification mark 102 is formed on the whole upper surface of the marker unit 100B by coating, or the like.

In this way, when the plurality of leaves 70 which constitute the leaf group 70G are disposed at the retracting end position P which is a predetermined reference position, gaps between adjacent identification marks 102 in the plate thickness direction T are formed to be larger than gaps between adjacent leaves 70 in the plate thickness direction T.

As in the first embodiment, a position of the identification mark 102 formed on the marker unit 100B is detected by the mark position detection unit 200. In this way, a position of each of the leaves 70 in the advancing and retracting direction may be detected.

Consequently, according to the multi-leaf collimator 60 and the radiation therapy device 20 of the second embodiment described above, the gaps between adjacent identification marks 102 in the plate thickness direction T may be formed to be larger than the gaps between adjacent leaves 70 in the plate thickness direction T since the plate thickness T11 of the marker unit 100B is smaller than the plate thickness T1 of the leaf 70. As a result, identification of the identification mark 102 on the marker unit 100B provided on the leaf 70 can be ensured and a position of the leaf 70 can be detected with high precision.

In addition, since the marker unit 100B is integrally formed with the upper edge portion 70a of the leaf 70, the marker unit 100B may be simultaneously formed by mechanical processing and the like when the leaf 70 is being manufactured. As a result, the marker unit 100B can easily be formed with high precision. In addition, the marker unit 100B may be suppressed from being deformed or folded due to a user's contact or the like since the marker unit 100B does not protrude from the rear edge portion 70d of the leaf 70 as the marker unit 100A in the first embodiment. As a result, identification of the marker unit 100B may be more ensured.

In addition, since the identification mark 102 is formed by coating or the like on the upper surface of the marker unit 100B which is a convex portion, the identification mark 102 to be identified by image processing may easily be formed in a predetermined size.

### (Third embodiment)

Next, a multi-leaf collimator and a radiation therapy device according to a third embodiment of the present invention will be described. Since only constitutions of marker units 100C and 100D are different in the third embodiment to be described below compared to the first embodiment, similar reference signs will be given to parts that are the same as in the first embodiment and overlapping descriptions thereof will be omitted.

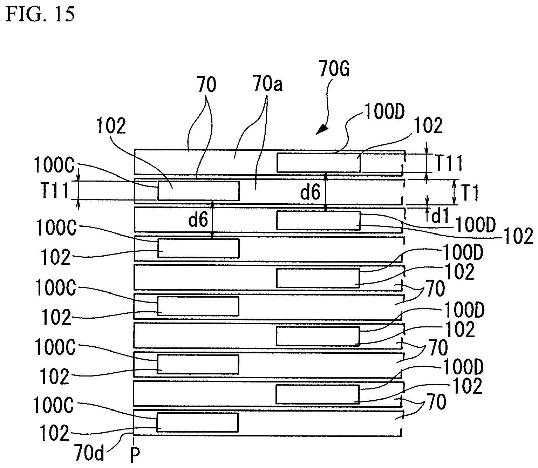

Fig. 15 is a plan view illustrating a constitution of an identification mark provided on each leaf according to a third embodiment of the present invention.

As illustrated in Fig. 15, in the leaf group 70G of the embodiment, the leaf 70 on which the marker unit 100C is formed at a side of the rear edge portion 70d of the upper edge portion 70a and the leaf 70 on which the marker unit 100D is formed at a side in front of the rear edge portion 70d are alternately disposed in the plate thickness direction T. In this way, when the rear edge portions 70d are aligned in the retracting end position P which is a predetermined reference position, the marker unit 100C and the marker unit 100D of adjacent leaves 70 in the plate thickness direction T are disposed at different positions in the advancing and retracting direction of the leaves 70.

As illustrated in Fig. 15, when the plurality of leaves 70 which constitute the leaf group 70G are disposed with the rear edge portions 70d aligned in the retracting end position P which is a predetermined reference position, gaps d6 between adjacent marker units 100C in the plate thickness direction T and gaps d6 between adjacent marker units 100D in the plate thickness direction T are formed to be larger than the gaps d1 between the leaves 70. Here, adjacent marker units 100C in the plate thickness direction T refer to the marker units 100C formed on leaves 70 which are two neighbors away from each other. Likewise, adjacent marker units 100D in the plate thickness direction T refer to the marker units 100D formed on leaves 70 which are two neighbors away from each other.

Like the marker unit 100B of the second embodiment, the marker units 100C and 100D are integrally formed on the upper edge portion 70a of each of the leaves 70 which constitute the leaf group 70G. The marker units 100C and 100D are formed such that the plate thickness T11 thereof in the plate thickness direction T is smaller than the plate thickness T1 of the leaf 70. The identification mark 102 is formed on the whole upper surface of the marker unit 100B by coating, or the like. The marker units 100C and 100D of the embodiment are disposed at the central portion of the leaf 70 in the plate thickness direction T.

Like in the first embodiment, positions of the identification marks 102 formed on the marker units 100C and 100D are detected by the mark position detection unit 200. In this way, a position of each of the leaves 70 in the advancing and retracting direction may be detected.

Consequently, according to the multi-leaf collimator 60 and the radiation therapy device 20 according to the third embodiment described above, since positions of the marker unit 100C and the marker unit 100D are different in the advancing and retracting direction of the leaves 70 between adjacent leaves 70 and 70 in the plate thickness direction T, identification of the identification marks 102 on the marker units 100C and 100D provided on the leaves 70 can be ensured and positions of the leaves 70 can be detected with high precision.

### (Modified example of the third embodiment)

Here, in the third embodiment, the marker units 100C and 100D are formed such that the plate thickness T11 thereof in the plate thickness direction T is smaller than the plate thickness T1 of the leaf 70. However, embodiments are not limited as this size relation between the plate thicknesses T1 and T11.

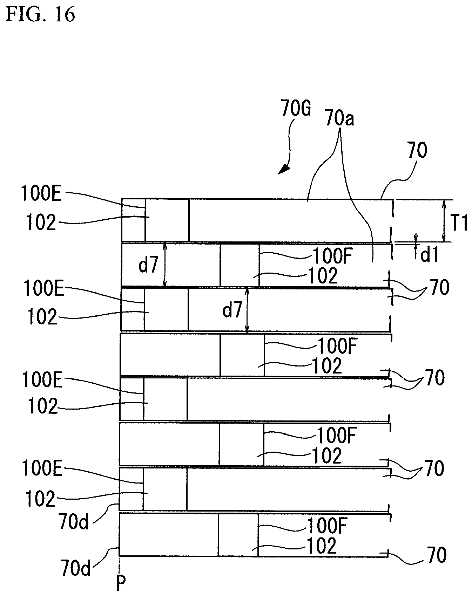

Fig. 16 is a plan view illustrating identification marks provided on a plurality of leaves according to a first modified example of the third embodiment.

As illustrated in Fig. 16, marker units 100E and 100F may be formed to have the same width as the plate thickness T1 of the leaf 70.

In a constitution of the first modified example of the third embodiment, the marker unit 100E and the marker unit 100F of adjacent leaves 70 and 70 in the plate thickness direction T are disposed in different positions in the advancing and retracting direction of the leaf 70. In this way, gaps d7 between the marker units 100E and between the marker units 100F adjacent in the plate thickness direction T are larger than the gaps d1 between the leaves 70.

Thus, identification of the identification marks 102 on the marker units 100E and 100F provided on the leaves 70 can be more ensured and positions of the leaves 70 can be detected with high precision.

### (Other modified example)

In addition, the present invention is not limited to each of the embodiments described above and includes various modifications made to each of the embodiments described above within the scope not departing from the gist of the present invention. That is, specific shapes, constitutions, etc. mentioned in each of the embodiments are merely examples and may be properly changed.

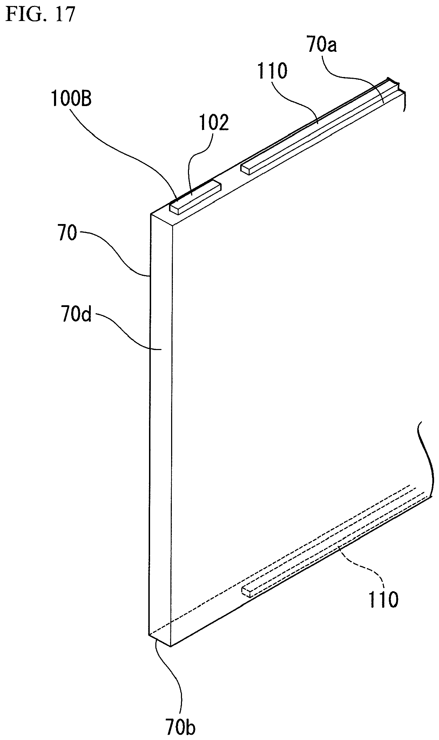

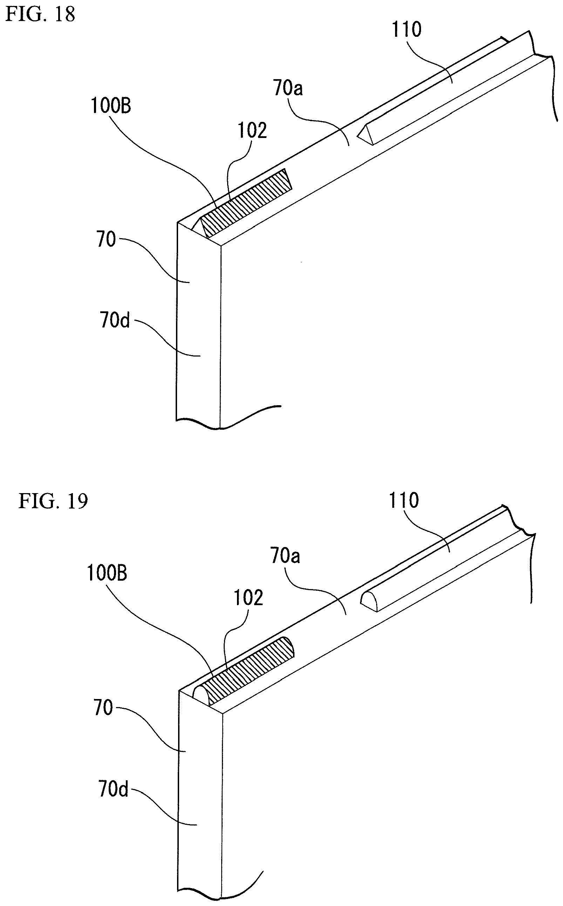

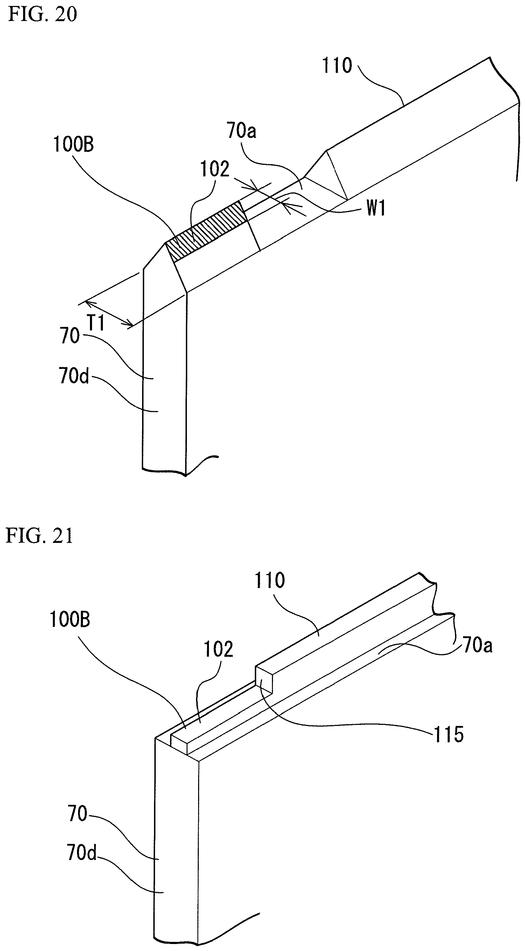

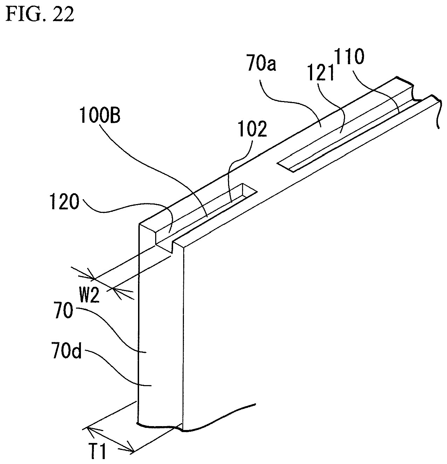

Fig. 17 is a perspective view illustrating a constitution of a leaf including an identification mark according to a first modified example of the second embodiment. Fig. 18 is a perspective view illustrating a constitution of a leaf including an identification mark according to a second modified example of the second embodiment. Fig. 19 is a perspective view illustrating a constitution of a leaf including an identification mark according to a third modified example of the second embodiment. Fig. 20 is a perspective view illustrating a constitution of a leaf including an identification mark according to a fourth modified example of the second embodiment. Fig. 21 is a perspective view illustrating a constitution of a leaf including an identification mark according to a fifth modified example of the second embodiment. Fig. 22 is a perspective view illustrating a constitution of a leaf including an identification mark according to a sixth modified example of the second embodiment.

For example, in the second embodiment, the leaf 70 on which the marker unit 100B is formed is guided by the rolling of the support rollers 66b.

On the other hand, in the first modified example illustrated in Fig. 17, a guide rail 110 is continuously formed in the same cross-sectional shape as the marker unit 100B on the upper edge portion 70a and the lower edge portion 70b of the leaf 70. The leaf 70 is guided along the guide rail 110 by the rolling of the support rollers 66b (refer to Fig. 6).

Here, since the guide rail 110 facilitates identification of the identification mark 102 on the marker unit 100B, the guide rail 110 may be formed apart from the marker unit 100B in the advancing and retracting direction.

In addition, the shape of the marker unit 100B is not limited to the rectangular parallelepiped shape and may have a different cross-sectional shape as in second to fourth modified examples illustrated in Figs. 18 to 21.

For example, in the second modified example illustrated in Fig. 18, the marker unit 100B and the guide rail 110 are formed to have a triangular cross-sectional shape, and the identification mark 102 is formed by coating, or the like on an inclined upper half portion at both sides of the top portion of the marker unit 100B.

In the third modified example illustrated in Fig. 19, the marker unit 100B and the guide rail 110 are formed to have a semicircular cross-sectional shape, and the identification mark 102 is formed by coating, and the like on a curved surface facing upward from the marker unit 100B.

In addition, as in the fourth modified example illustrated in Fig. 20, when the marker unit 100B and the guide rail 110 are formed to have a triangular cross-sectional shape whose width becomes gradually smaller upward, the width of a bottom side may be formed to be the same as the plate thickness of the leaf 70.

In this case, the identification mark 102 is formed by coating, and the like only within a predetermined range around the top portion of the marker unit 100B. In this way, a width W1 of the identification mark 102 becomes smaller than the plate thickness T1 of the leaf 70. Thus, as in the first and second embodiments, identifiability of the identification mark 102 is improved and a position of the leaf 70 can be detected with high precision.

Here, with respect to the second to fourth modified examples illustrated in Figs. 18, 19, and 20, the marker unit 100B and the guide rail 110 may have different cross-sectional shapes from each other.

In addition, as in a fifth modified example illustrated in Fig. 21, the guide rail 110 may be continuously provided on the marker unit 100B. Here, the heights of the marker unit 100B and the guide rail 110 are different from each other. In this way, a stepped portion 115 is formed between the marker unit 100B and the guide rail 110. A boundary between the identification mark 102 on the marker unit 100B and other portions becomes clear by the stepped portion 115, and the identifiability of the identification mark 102 can be improved.

In addition, as in a sixth modified example illustrated in Fig. 22, a groove 120 may be formed on the upper edge portion of the leaf 70, and the groove 120 may be the marker unit 100B. In addition, the identification mark 102 is formed by coating, and the like on a bottom surface of the groove 120.

In this case, a groove width W2 of the groove 120 is made smaller than the plate thickness T1 of the leaf 70. In this way, as in the first embodiment and the second embodiment, the identifiability of the identification mark 102 is improved and a position of the leaf 70 can be detected with high precision.

In addition, even in the sixth modified example illustrated in Fig. 22, the guide rail 110 formed of a groove 121 having the same cross-sectional shape as the marker unit 100B may be provided. In this case, outer circumferential portions of the support rollers 66b of the slide support member 66 are engaged inside the groove 121.

In addition, in the first embodiment to the third embodiment (also including the modified examples of each), the marker units 100A to 100F are provided at a side of the rear edge portion 70d of the leaf 70. However, the marker units 100A to 100F may also be provided at a side of the front edge portion 70c.

In addition, in each of the embodiments described above, when the plurality of leaves 70 are disposed at a predetermined reference position, the gaps d2 to d7 between adjacent marker units 100A to 100F in the plate thickness direction T are formed to be larger than the gaps d1 between the leaves 70. In addition, in each of the embodiments, the retracting end position P at which the rear edge portion 70d of each of the leaves 70 comes in contact with the stopper 68 (refer to Figs. 4 and 6) is described as an example of the reference position. However, embodiments are not limited to this constitution. For example, an advancing end position at which each of the leaves 70 is maximally advanced to a side of the front edge portion 70c may be the reference position, and a central position between the retracting end position P and the advancing end position may be the reference position.

In addition, cases in which all of the marker units 100A to 100D are disposed at the central portion of the leaf 70 in the plate thickness direction have been described. However, the arrangement of the marker units 100A to 100D is not limited thereto as long as the gaps between adjacent marker units in the plate thickness direction T are larger than the gaps between adjacent leaves 70 in the plate thickness direction T.

In addition, the identification mark 102 may be formed on the marker units 100A to 100D by applying white paint, pigment, a seal, etc.

In addition, the constitution of the first modified example and the second modified example of the first embodiment in which the leaves 70 have several types of thicknesses may also be applied to the second embodiment, each of the modified examples of the second embodiment, and the third embodiment.

In addition, the cross-sectional shape of the marker unit 100B described from the first modified example to the sixth modified example of the second embodiment may also be applied to the marker units 100C and 100D of the third embodiment. In addition, the guide rail 110 may also be provided on the leaf 70 of the third embodiment.

### [Industrial Applicability]

By forming gaps between adjacent identification marks in a thickness direction to be larger than gaps between adjacent leaves in the thickness direction, identification of an identification mark provided on a leaf can be more ensured and a position of the leaf can be promptly detected with high precision.

### [Reference Signs List]

10 Radiation therapy system
11 Treatment planning device
12 Control device (control unit)
20 Radiation therapy device
21 Ring frame
21a Lower end portion
22 Travelling gantry
22a Inner circumferential surface
23 Sensor array
24 Radiation irradiation device
25 Rotation shaft
26A, 26B X-ray source
27A, 27B Sensor array
28 Couch
28a Upper surface
29 Couch driving device
51 Electron beam acceleration device
52 X-ray target
53 First collimator
53h Through-hole
54 Flattening filter
54a Protrusion
55 Second collimator
55h Through-hole
60 Multi-leaf collimator
61 Frame
61a Upper surface portion
61b Lower surface portion
61c, 61d Side surface portion
62 Leaf accommodation unit
64 Opening
65 Base plate
66 Slide support member
66a Shaft
66b Support roller
67 Opening
68 Stopper
70 Leaf
70G Leaf group
70a Upper edge portion (end surface)
70b Lower edge portion
70c Front edge portion
70d Rear edge portion
71, 72 Slit
71a, 72b Upper side portion
71b Lower side portion
73 Rack gear
90 Driving device (driving mechanism)
91 Motor
91a Housing
92 Rotary encoder
94 Cover
95 Shaft
96 Pinion gear
97 Bearing
100A to 100F Marker unit
101 Diagnostic X-rays
102 Identification mark
110 Guide rail
115 Stepped portion
120, 121 Groove
200 Mark position detection unit
201 Mirror
202 Illumination
203 Camera
204 Image processing unit
205 Driving control unit
B Patient
C0 Isocenter
C1, C2 Central axis
d1 to d7 Gap
P Retracting end position (reference position)
S0 Electron beam
S1 Radiation
S2 Radiation
Sr Therapeutic radiation

## Claims

1. A multi-leaf collimator (60) which limits a radiation field of radiation, the multi-leaf collimator (60) comprising:
a plurality of leaves (70) each formed in a shape of a plate and aligned in a thickness direction (T) of the leaf (70), the thickness direction (T) being perpendicular to the plate surface of the leaf (70);
a driving mechanism (90) configured to advance and retract each of the plurality of leaves (70) with respect to the radiation; and
an identification mark (102) provided on each of the plurality of leaves (70) and identifiable at least from a direction (C2) perpendicular to both the advancing and retracting direction (W) of the leaves (70) and the thickness direction (T) of the leaf (70),
wherein, when the plurality of leaves (70) are disposed in a preset reference position (P), gaps (d2) between the adjacent identification marks (102) in the thickness direction (T) are formed to be larger than gaps (d1) between the adjacent leaves (70) in the thickness direction (T), and
wherein a width (T11) of the identification mark (102) in the thickness direction (T) is formed to be smaller than a thickness (T1) of the leaf (70) including the identification mark (102),
**characterized in that**
the plurality of leaves (70) are constituted of various types of leaves having different thicknesses, and
the number of types of widths (T11) of the identification mark (102) is equal to or less than the number of types of thicknesses (T1) of the leaves (70), and
the identification marks (102) of the adjacent leaves (70) in the thickness direction (T) are disposed at different positions in the advancing and retracting direction (W).

2. The multi-leaf collimator (60) according to claim 1, wherein the identification mark (102) is formed at an end surface (70a) in a direction (C2) perpendicular to both the advancing and retracting direction (W) and the thickness direction (T) of the leaf (70).

3. The multi-leaf collimator (60) according to claim 2, wherein the end surface (70a) includes at least one of a convex portion (100B) and a groove portion (100B), and
wherein the identification mark (102) is formed on at least one of the convex portion (100B) and the groove portion (100B).

4. A radiation therapy device (11) comprising:
the multi-leaf collimator (60) according to any one of claims 1 to 3;
a mark position detection unit (200) configured to detect a position of the identification mark (102);
a control unit (12) configured to control the driving mechanism (90) based on the position of the identification mark (102) detected by the mark position detection unit (200); and
a radiation irradiation device (20) configured to radiate radiation.

## Patentansprüche

1. Ein Mehrblattkollimator (60), der ein Strahlungsfeld von Strahlung begrenzt, wobei der Mehrblattkollimator (60) aufweist:
eine Mehrzahl von Blättern (70), die jeweils plattenförmig ausgestaltet sind und in einer Dickenrichtung (T) des Blattes (70) parallel ausgerichtet sind, wobei die Dickenrichtung (T) senkrecht zu der Plattenoberfläche des Blatts (70) verläuft;
einen Antriebsmechanismus (90), der dazu ausgelegt ist, jedes aus der Vielzahl von Blättern (70) in Bezug auf die Strahlung vor- und zurückzuziehen; und
eine Identifikationsmarkierung (102), die auf jeder der Vielzahl von Blättern (70) vorgesehen ist und zumindest aus einer Richtung (C2) senkrecht zu der Vor- und Rückzugsrichtung (W) der Blätter (70) und der Dickenrichtung (T) des Blattes (70) erkennbar ist,
wobei, wenn die Vielzahl von Blättern (70) in einer vorgegebenen Referenzposition (P) angeordnet sind, Spaltbreiten (d2) zwischen den benachbarten Identifikationsmarken (102) in der Dickenrichtung (T) größer ausgestaltet sind als Spaltbreiten (d1) zwischen den benachbarten Blättern (70) in der Dickenrichtung (T), und
wobei eine Breite (T11) der Identifikationsmarkierung (102) in der Dickenrichtung (T) kleiner ausgestaltet ist als eine Dicke (T1) des Blattes (70), welches die Identifikationsmarkierung (102) umfasst,
**dadurch gekennzeichnet, dass**
die Mehrzahl von Blättern (70) durch verschiedene Arten von Blättern mit unterschiedlicher Dicke gebildet ist, und
die Anzahl der Arten von Breiten (T11) der Identifikationsmarkierung (102) kleiner oder gleich der Anzahl der Arten der Dicken (T1) der Blätter (70) ist, und
die Identifikationsmarkierungen (102) der benachbarten Blätter (70) in der Dickenrichtung (T) in der Vor- und Rückzugsrichtung (W) in unterschiedlichen Positionen angeordnet sind.

2. Mehrblattkollimator (60) nach Anspruch 1, wobei die Identifikationsmarkierung (102) an einer Endfläche (70a) in einer Richtung (C2) senkrecht zu der Vor- und Rückzugsrichtung (W) und der Dickenrichtung (T) des Blattes (70) ausgebildet ist.

3. Mehrblattkollimator (60) nach Anspruch 2, wobei die Endfläche (70a) mindestens einen konvexen Abschnitt (100B) und einen Rillenabschnitt (100B) aufweist und wobei die Identifikationsmarkierung (102) an mindestens einem der Abschnitte aus dem konvexen Abschnitt (100B) und dem Rillenabschnitt (100B) ausgebildet ist.

4. Strahlentherapiegerät, umfassend:
den Mehrblattkollimator (60) nach einem der Ansprüche 1 bis 3; eine Markierungspositions-Erfassungseinheit (200), die dazu ausgelegt ist, eine Position der Identifikationsmarkierung (102) zu erfassen;
eine Steuereinheit (12), die dazu ausgelegt ist, den Antriebsmechanismus (90) auf der Grundlage der Position der Identifikationsmarkierung (102) zu steuern, die durch die Markierungspositions-Erfassungseinheit (200) erfasst wird; und
eine Strahlungsbestrahlungseinrichtung (20), die dazu ausgelegt ist, die Strahlung zu emittieren.

## Revendications

1. Un collimateur multifeuilles (60) qui limite un champ de rayonnement, le collimateur multifeuilles (60) comprenant :
une pluralité de feuilles (70) alignées dans le sens d'épaisseur (T) de la feuille (70), le sens d'épaisseur (T) étant perpendiculaire à la surface plate de la feuille (70) ;
un mécanisme d'entraînement (90) configuré pour avancer et rétracter chacune de la pluralité de feuilles (70) par rapport au rayonnement ; et
une marque d'identification (102) prévue sur chacune de la pluralité de feuilles (70) et identifiable au moins par une direction (C2) perpendiculaire à la fois à la direction d'avancement et de retrait (W) des feuilles (70) et au sens de l'épaisseur (T) de la feuille (70),
dans lequel, lorsque la pluralité de feuilles (70) est disposée dans une position de référence (P) prédéterminée, les interstices (d2) entre les marques d'identification (102) adjacentes dans le sens de l'épaisseur (T) sont formés plus grands que les interstices entre les feuilles (70) adjacentes dans le sens de l'épaisseur (T) et,
dans lequel une largeur (T11) de la marque d'identification (102) dans le sens de l'épaisseur (T) est formée inférieure à une épaisseur (T1) de la feuille (70) incluant la marque d'identification (102),
**caractérise en ce que**
la pluralité de feuilles (70) est constituée de différents types de feuilles (70) ayant des épaisseurs différentes, et
le nombre de types de largeurs (T11) de la marque d'identification (102) est égal ou inférieur au nombre de types d'épaisseurs (T1) des feuilles (70) et,
les marques d'identification (102) des feuilles (70) adjacentes dans le sens de l'épaisseur (T) sont disposées dans des positions différentes dans la direction d'avancement et de retrait (W).

2. Collimateur multifeuilles (60) selon la revendication 1,
dans lequel la marque d'identification (102) est formée à une surface d'extrémité (70a) dans une direction (C2) perpendiculaire à la direction d'avancement et de retrait (W) et à la direction de l'épaisseur (T) de la feuille (70).

3. Collimateur multifeuilles (60) selon la revendication 2, dans lequel la surface d'extrémité (70a) comprend au moins une partie convexe (100B) et une partie de rainure (100B), et
dans lequel la marque d'identification (102) est formée sur au moins une partie choisie parmi la partie convexe (100B) et la partie de rainure (100B).

4. Dispositif de radiothérapie comprenant :
le collimateur multifeuilles (60) selon l'une quelconque des revendications 1 à 3 ;
une unité de détection de position de marque (200) configurée pour détecter une position de la marque d'identification (102) ;
une unité de commande (12) configurée pour commander le mécanisme d'entraînement (90) en fonction de la position de la marque d'identification (102) détectée par l'unité de détection de position de marque (200); et
un dispositif d'irradiation par rayonnement (20) configuré pour rayonner le rayonnement.
